# EUROPÄISCHE PATENTANMELDUNG

(11) **EP 0 715 857 A2**
(43) Veröffentlichungstag der Anmeldung: **12.06.1996**
(21) Anmeldenummer: 95118095.9
(22) Anmeldetag: 17.11.1995
(51) Int. Cl.: A61K 47/18

(54) **Pharmazeutische, oral anwendbare Zubereitungen enthaltend als Zerfallbeschleuniger eine wasserlösliche Aminosäure**

(30) Priorität: 10.12.1994 DE 4444051
(71) Anmelder: RHONE-POULENC RORER GMBH, 50792 Köln (DE)
(72) Erfinder: Gajdos, Benedikt, Dr., D-50767 Köln (DE); Dürr, Manfred, Dr., D-50129 Bergheim Glessen (DE)
(74) Vertreter: Döring, Wolfgang, Dr. Ing.

(57) **Zusammenfassung**

Es wird eine pharmazeutische, oral anwendbare Zubereitung beschrieben, wobei die feste Zubereitung mindestens einen Wirkstoff, mindestens ein Sprengmittel sowie mindestens einen pharmazeutisch üblichen Zuschlagstoff aufweist. Desweiteren enthält die Zubereitung mindestens einen, den Zerfall der Zubereitung im Mund oder in einer Flüssigkeit beschleunigenden Inhaltsstoff, wobei dieser Inhaltsstoff eine wasserlösliche Aminosäure, ein wasserlösliches Aminosäurederivat und/oder ein wasserlösliches Salz einer Aminosäure ist.

## Beschreibung

Die vorliegende Erfindung betrifft eine pharmazeutische, oral anwendbare Zubereitung mit den Merkmalen des Oberbegriffs des Patentanspruchs 1.

Feste, oral anwendbare pharmazeutische Zubereitungen, die mindestens einen Wirkstoff, mindestens ein Sprengmittel sowie mindestens einen pharmazeutisch üblichen Zuschlagsstoff aufweisen, sind seit langem bekannt und im Handel erhältlich. Abhängig von dem jeweiligen Wirkstoff bzw. dem Wirkstoffgemisch werden diese bekannten Zubereitungen als Tablette, Granulat oder Pulver zur Vorbeugung und/oder zur Behandlung von Schmerzzuständen aller Art, insbesondere zur Behandlung von Kopfschmerzen, Rheumaschmerzen, Gliederschmerzen, Migräne, Zahnschmerzen, sowie zur Behandlung von rheumatischen Erkrankungen, Gicht, nicht rheumatischen schmerzhaften Schwellungen und/oder Entzündungen angewendet. Hierfür ist es in der Regel erforderlich, daß der Patient die entsprechende Tablette oder das Granulat unzerkaut schluckt, was insbesondere bei größeren Tabletten schwierig ist, oder vor dem Verschlucken zerkaut, was vielfach zu einem Verkleben von Bestandteilen der Tablette bzw. des Granulates im Zahn- und/oder Gaumenbereich des Mundes führt. Diese verklebten Bestandteile lassen sich nur schwer entfernen und setzen darüber hinaus noch im Laufe der Zeit den Wirkstoff frei, was letztendlich dann einen äußerst unangenehmen Geschmack hervorruft.

Um die zuvor beschriebenen Probleme beim Einnehmen der bekannten pharmazeutischen Zubereitungen zu umgehen, sind speziell hergestellte Tabletten bekannt, bei denen der Zerfall im Mund oder in einer entsprechenden Flüssigkeit relativ schnell und zwangsläufig erfolgt, so daß hierbei ein Zerkauen der bekannten Tablette, die üblicherweise auch als Lyoc-Tablette bezeichnet wird, im Mund entfallen kann. Derartige spezielle und bekannte pharmazeutische Zubereitungen weisen jedoch den Nachteil auf, daß sie im Laufe ihrer Herstellung, während ihres Transportes zum Patienten oder bei ihrer Anwendung beim Patienten leicht und unerwünscht beschädigt werden können, so daß dementsprechend ihre Verwendung ebenfalls eingeschränkt ist.

Der vorliegenden Erfindung liegt die Aufgabe zugrunde, eine oral anwendbare, feste Zubereitung der angegebenen Art zur Verfügung zu stellen, die einerseits eine besonders hohe Haltbarkeit und andererseits bei ihrer Anwendung sehr schnell zerfällt.

Diese Aufgabe wird erfindungsgemäß durch eine pharmazeutische Zubereitung mit den kennzeichnenden Merkmalen des Patentanspruchs 1 gelöst.

Die erfindungsgemäß pharmazeutische und oral anwendbare feste Zubereitung weist mindestens einen Wirkstoff, mindestens ein Sprengmittel sowie mindestens einen pharmazeutisch üblichen Zuschlagsstoff auf. Darüber hinaus enthält die erfindungsgemäße Zubereitung desweiteren mindestens einen, den Zerfall der Zubereitung im Mund oder in einer Flüssigkeit beschleunigenden Inhaltsstoff, wobei dieser Inhaltsstoff eine wasserlösliche Aminosäure, ein wasserlösliches Aminosäurederivat und/oder ein wasserlösliches Salz einer Aminosäure ist.

Überraschend konnte festgestellt werden, daß die erfindungsgemäße Zubereitung aufgrund des zuvor genannten mindestens einen Inhaltsstoffes (wasserlösliche Aminosäure, wasserlösliches Aminosäurederivat und/oder wasserlösliches Salz einer Aminosäure) eine sehr hohe Zerfallsrate, d.h. somit zeitlich gesehen einen sehr schnellen Zerfall dann besitzt, wenn die erfindungsgemäße Zubereitung mit Speichel oder einer geeigneten Flüssigkeit, so insbesondere Wasser, in Kontakt kommt. Dies wiederum führt dazu, daß die erfindungsgemäße Zubereitung im Mund sehr schnell zerfällt, ohne daß es dabei erforderlich ist, die erfindungsgemäße Zubereitung durch Kauen zu zerkleinern. Folglich treten bei der erfindungsgemäßen Zubereitung nicht die Probleme auf, wie diese vorstehend bei den bekannten Zubereitungen, auch bei solchen Zubereitungen, die ein Sprengmittel enthalten, vorhanden sind, d.h. Bestandteile der erfindungsgemäßen Zubereitung setzen sich nicht in schwer zugänglichen Bereichen des Mundes ab und verursachen demnach auch kein unerwünschtes Verkleben auf der Zunge und/oder am Gaumen bzw. im Bereich der Zähne, so daß es dementsprechend bei der erfindungsgemäßen Zubereitung auch nicht dazu kommen kann, daß der jeweilige Patient bei Anwendung der erfindungsgemäßen Zubereitung einen bitteren Geschmack, hervorgerufen durch eine Freisetzung des Wirkstoffes, empfindet. Weiterhin wird durch den zuvor genannten und den Zerfall der Zubereitung im Mund oder in einer Flüssigkeit beschleunigenden Inhaltsstoff (wasserlösliche Aminosäure, wasserlösliches Aminosäurederivat und/oder wasserlösliches Salz einer Aminosäure) die Festigkeit der erfindungsgemäßen Zubereitung nicht verschlechtert, so daß es auch bei der erfindungsgemäßen Zubereitung nicht dazu kommen kann, daß die erfindungsgemäße Zubereitung bei ihrer Herstellung, dem Transport und ihrer Anwendung unerwünscht beschädigt wird, so daß dementsprechend die Reklamationsrate bei der erfindungsgemäßen Zubereitung besonders niedrig ist. Auch läßt sich die erfindungsgemäße Zubereitung nach konventionellen Herstellungstechniken besonders preisgünstig produzieren, während dies bei der bekannten und zuvor genannten Lyoc-Tabletten nicht der Fall ist.

Die zuvor angesprochene hohe Zerfallsrate der erfindungsgemäßen Zubereitung wird auf einen synergistischen Effekt des mindestens einen Sprengmittels mit dem Inhaltsstoff (wasserlösliche Aminosäure, wasserlösliches Aminosäurederivat und/oder wasserlösliches Salz einer Aminosäure) zurückgeführt.

Grundsätzlich kann die erfindungsgemäße Zubereitung jede wasserlösliche Aminosäure, jedes wasserlösliche Aminosäurederivat und/oder jedes wasserlösliches Salz einer Aminosäure enthalten, sofern sichergestellt ist, daß die zuvor genannten Inhaltsstoffe, die den Zerfall der erfindungsgemäßen Zubereitung im Mund oder in einer Flüssigkeit beschleunigen, untoxisch sind und keine Wechselwirkungen mit dem mindestens einen Wirkstoff eingehen. Besonders geeignet ist es, wenn die erfindungsgemäße Zubereitung als den Zerfall der Zubereitung beschleunigenden Inhaltsstoff, Glycin, ein Glycinderivat und/oder ein Glycinsalz enthält, wobei unter den Begriff Glycinderivat insbesondere Ester, vorzugsweise von C₁-C₄-Alkoholen, und/oder Amide von Glycin, vorzugsweise von C₁-C₁₀-Carbonsäuren, und unter den Begriff Glycinsalze vorzugsweise wasserlösliche Alkali- und/oder Erdalkalisalze sowie entsprechende Ammoniumsalze fallen. Diese zuvor beschriebenen Ausführungsformen der erfindungsgemäßen Zubereitung, die als Inhaltsstoff Glycin, ein Glycinderivat, ein Glycinsalz und/oder eine Mischung davon enthalten, sind toxikologisch völlig unbedenklich, wobei aufgrund des relativ geringen Preises der zuvor genannten und auf Glycin basierenden Inhaltsstoffe diese Ausführungsform der erfindungsgemäßen Zubereitung besonders preiswert herstellbar ist.

Eine andere Ausführungsform der erfindungsgemäßen pharmazeutischen Zubereitung weist zusätzlich zu den zuvor abgehandelten Inhaltsstoffen auf Glycin-Basis oder anstelle der zuvor genannten Inhaltsstoffe auf Glycin-Basis solche den Zerfall der Zubereitung beschleunigenden Inhaltsstoffe auf, die aus der Gruppe, bestehend aus Prolin, Hydroxyprolin und/oder Lysin sowie deren Salze und/oder Derivate, ausgewählt sind. Hierbei umfaßt der Begriff Salze und der Begriff Derivate die zuvor beim Glycin genannten Salze bzw. Derivate, wobei jedoch zusätzlich hierbei noch beim Prolin bzw. beim Hydroxyprolin die Möglichkeit besteht, den Pyrrolidin-Ring entsprechend zu substituieren, insbesondere zu halogenieren und/oder am Pyrrolidin-Ring eine zusätzliche NH₂-Gruppe, NO₂-Gruppe und/oder eine SO₃H-Gruppe vorzusehen. Ebenso kann an den nicht substituierten CH₂-Gruppen des Lysins einer oder mehrere der zuvor genannten Substituenten angeordnet werden.

Bezüglich der in der erfindungsgemäßen Zubereitung enthaltenen und vorstehend detailliert beschriebenen Inhaltsstoffen, die den Zerfall der erfindungsgemäßen Zubereitung im Mund oder einer Flüssigkeit beschleunigen, ist festzuhalten, daß die Inhaltsstoffe in einer solchen Konzentration in der erfindungsgemäßen Zubereitung enthalten sind, daß die Zubereitung im Mund bzw. in einer ausgewählten Flüssigkeit innerhalb von einer Sekunde bis sechzig Sekunden, vorzugsweise innerhalb von einer Sekunde bis dreißig Sekunden, zerfällt.

Abhängig von dem jeweiligen Wirkstoff sowie den üblichen Zuschlagsstoffen sowie der Aufmachung der festen erfindungsgemäßen Zubereitung variiert die Konzentration des Inhaltsstoffes bzw. des Gemisches der Inhaltsstoffes in der erfindungsgemäßen Zubereitung zwischen 1 Gew.% und 90 Gew.%, insbesondere zwischen 20 Gew.% und 70 Gew.%, bezogen auf die anwendungsfertige Zubereitung.

Als Sprengmittel sind vorzugsweise in der erfindungsgemäßen Zubereitung Stärke, eine Stärkederivat, Cellulose, ein Cellulosederivat, Alginsäure, ein Alginsäurederivat, Casein, ein Caseinderivat und/oder ein unlösliches Polyvinylpyrrolidon (Crosspolyvidone) enthalten. Konkret handelt es sich bei der zuvor genannten Stärke insbesondere um Mais- oder Kartoffelstärke, bei dem zuvor genannten Stärkederivat insbesondere um modifizierte Stärke und/oder Natriumcarboxymethylstarke, bei dem zuvor genannten Cellulosederivat, insbesondere um Carboxymethylcellulose und/oder Natriumcarboxymethylcellulose. Desweiteren eignen sich als Sprengmittel quervernetztes Casein, Natriumalginat sowie in Speichel und/oder Wasser unlösliches Polyvinylpyrrolidon, wobei das letztgenannte Produkt auch unter der Bezeichnung Kollidon CL und Polyplasdone XL im Handel erhältlich ist.

Bezüglich der Konzentration des in der erfindungsgemäßen Zubereitung enthaltenen Sprengmittels ist festzuhalten, daß diese zwischen 1 Gew.% und 50 Gew.%, insbesondere zwischen 3 Gew.% und 20 Gew.%, bezogen auf die anwendungsfertige Zubereitung, variiert.

Eine besonders vorteilhafte Weiterbildung der zuvor beschriebenen Ausführungsform der erfindungsgemäßen Zubereitung sieht vor, daß hierbei die Zubereitung den mindestens einen Wirkstoff in Form von gleichmäßig in der festen Zubereitung verteilten Wirkstoffpartikeln aufweist. Eine derartige Weiterbildung der erfindungsgemäßen Zubereitung besitzt neben einer ausgezeichneten Haltbarkeit, einer besonders schnellen Zerfallsrate auch eine besondere hohe pharmazeutische Wirksamkeit. Dies hängt damit zusammen, daß nach Zerfall der erfindungsgemäßen Zubereitung die dann in den Magen bzw. Darm gelangenden einzelnen Wirkstoffpartikel eine relativ große Oberfläche besitzen, so daß sie dementsprechend schnell die erwünschte therapeutische Wirkung hervorrufen können.

Bezüglich des in der erfindungsgemäßen Zubereitung enthaltenen Wirkstoffes bzw. des Wirkstoffgemisches ist festzuhalten, daß es sich hierbei grundsätzlich um übliche pharmazeutische Wirkstoffe bzw. Wirkstoffgemische handelt. Vorzugsweise weist die erfindungsgemäße Zubereitung jedoch solche Wirkstoffe bzw. Wirkstoffgemische auf, die üblicherweise in den an sich bekannten Krebsmitteln, Antibiotika, Antipyretika, Immunstimulatoren, Immunsuppressiva, Entzündungshemmern, Antiepileptika, Mitteln zur Behandlung von zerebralen Erkrankungen, Antihistaminika, Diuretika, Blutdrucksenkern, Antidiabetika, Muskelrelaxantien, Ulkushemmern, Antidepressiva, Antallergika, herzstimulierenden Mitteln, Vasodilatatoren, Gerinnungshemmern, Husten- und/oder Erkältungsmitteln sowie Vitaminen enthalten sind.

Besonders bevorzugt im Sinne der vorliegenden Erfindung ist eine Zubereitung, die einen Wirkstoff mit entzündungshemmenden, antirheumatischen bzw. analgetischen Eigenschaften aufweist. Vorzugsweise ist dann der Wirkstoff bzw. das Wirkstoffgemisch auf der Basis einer Pyrazolonverbindung, einer Salicylsäureverbindung, einer Phenylbutanzonverbindung, einer Paracetamolverbindung, einer Antranilsäureverbindung, einer Arylessigsäureverbindung und/oder einer Arylpropionsäureverbindung aufgebaut.

Konkret zählen zu der erfindungsgemäßen pharmazeutischen Zubereitung solche Zubereitungen, die als Wirkstoff bzw. Wirkstoffgemisch Ibuprofen, Ketoprofen, Naproxen, Tolmetin, Furabiprofen, Indomethacin, Mefenaminsäure, Flufenaminsäure, Paracetamol, Acetylsalicylsäure, Alclofenac, Ketoprofen, Ibuprofen und/oder Diclofenac-Natrium enthalten, wobei die zuerst genannten Wirkstoffe dann vorliegen, wenn die erfindungsgemäße Zubereitung als Analgetikum angewendet werden soll, und die zuletzt genannten vier Wirkstoffe dann in der erfindungsgemäßen Zubereitung enthalten sind, wenn die erfindungsgemäße Zubereitung als Antirheumatikum eingesetzt wird.

Bezüglich der Konzentration des Wirkstoffes und vorzugsweise der zuvor genannten Wirkstoffe in der erfindungsgemäßen Zubereitung ist festzuhalten, daß diese Konzentration des Wirkstoffes bzw. Wirkstoffgemisches zwischen 1 Gew.% und 50 Gew.%, insbesondere zwischen 15 Gew.% und 40 Gew.%, jeweils bezogen auf die anwendungsfertige Zubereitung, variiert.

Insbesondere dann, wenn die erfindungsgemäße Zubereitung als Wirkstoff einen hydrophilen Wirkstoff enthält, empfiehlt es sich, diesen hydrophilen Wirkstoff mit einer hydrophoben Beschichtungsschicht zu versehen und/oder den hydrophilen Wirkstoff in einer hydrophoben Matrix einzubetten. Diese Ausführungsform der erfindungsgemäßen Zubereitung weist nicht nur die bereits vorstehend genannten Vorteile (hohe Zerfallsrate, ausreichende Härte und Beständigkeit) auf, sondern zeichnet sich insbesondere auch noch dadurch aus, daß beim Zerfall der erfindungsgemäßen Zubereitung im Mund der Wirkstoff bzw. die konkreten Wirkstoffpartikel durch die hydrophobe Beschichtungsschicht bzw. die hydrophobe Matrix daran gehindert wird bzw. werden, sich zu größeren Agglomeraten zusammenzuballen, was der erwünschten Feinverteilung des Wirkstoffes entgegenstehen würde. Desweiteren wird durch eine derartige hydrophobe Beschichtungsschicht bzw. hydrophobe Matrix erreicht, daß ein möglicherweise vom Wirkstoff ausgehender bitterer oder unangenehmer Geschmack unterdrückt wird.

Die zuvor erwähnte Unterdrückung des bitteren und/oder unangenehmen Geschmackes des Wirkstoffes kann auch dadurch erreicht werden, daß die erfindungsgemäße Zubereitung einen Wirkstoff und/oder ein Wirkstoffgemisch aufweist, der bzw. das nicht mit einer hydrophoben Beschichtungsschicht versehen bzw. in einer hydrophoben Matrix eingebettet ist, sondern stattdessen größere, zuvor kompaktierte Wirkstoffteilchen aufweist. Durch ein derartige Kompaktieren, insbesondere durch ein Zusammenpressen, der Wirkstoffteilchen zu größeren Agglomeraten wird dann die zur Verfügung stehende Oberfläche im Vergleich zu konkreten Wirkstoffpartikeln verkleinert, so daß dementsprechend der Auflösevorgang des Wirkstoffes im Mund verzögert wird, was wiederum einen unangenehmen oder bitteren Geschmack unterdrückt.

Grundsätzlich können zu der zuvor angesprochenen Beschichtung bzw. Einbettung des Wirkstoffes bzw. des Wirkstoffgemisches in einer hydrophoben Matrix alle Beschichtungsmittel bzw. Einbettungsmittel ausgewählt werden, die einerseits die erwünschte Hydrophobierung des Wirkstoffes bzw. des Wirkstoffgemisches sicherstellen und andererseits toxisch unbedenklich sind. Hierfür kommen insbesondere Beschichtungsschichten bzw. Einbettungsmittel in Frage, die aus der Gruppe, bestehend aus Schellack, Stearinsäure, Gelatine, Zein, Gummi arabicum, Cellulosederivate, polymere Acrylsäurederivate und/oder polymere Vinylacetate ausgewählt sind. Konkret sind hier Methylcellulose, Ethylcellulose, Hydroxypropylcellulose, Hydroxypropylmethylcellulose, Celluloseacetat-Phthalat, Hydroxypropylmethylcellulosephthalat sowie polymere Acrylsäurederivate, insbesondere Copolymerisate aus Methacrylsäure und Estern von Methacrylsäuren, Acrylsäureethyl-Methacrylsäuremethylester-Copolymerisate, Methacrylsäure-Acrylsäuremethylester-Copolymerisate, Acryl- und Methacrylsäureester-Copolymerisate mit Trimethylammoniummethacrylat, Copolymerisate von Dimethylaminomethacrylsäure und neutralen Methacrylsäureestern, Vinylpyrrolidon-Vinylacetat-Copolymerisate, Polyvinylacetate, Polyvinylpyrrolidon, sowie Polyethylenglycol zu nennen.

Bezüglich der Mengen der zuvor genannten Beschichtungs- bzw. Einbettungsmittel ist festzuhalten, daß diese Menge der Beschichtungs- bzw. Einbettungsmittel abhängig von der Konzentration des jeweiligen hydrophilen Wirkstoffes bzw. des hydrophilen Wirkstoffgemisches zwischen 1 Gew.% und 10 Gew.%, bezogen auf die anwendungsfertige erfindungsgemäße Zubereitung, variiert.

Desweiteren sind in der erfindungsgemäßen Zubereitung pharmazeutisch übliche Zuschlagsstoffe enthalten. Hierunter fallen insbesondere Füllmittel, Bindemittel, Gleitmittel, Feuchthaltemittel, Absorptionsmittel, Antistatika, Farbstoffe und Geschmacksstoffe.

Insbesondere weist die erfindungsgemäße Zubereitung zwischen 0 Gew.% und 60 Gew.% pharmazeutisch übliche Füll- und Bindemittel, jeweils zwischen 0 Gew.% und 10 Gew.% Povidon, Zucker, Zuckeralkohole und/oder Polyethylenglycol sowie eine übliche Konzentration an Geschmacksstoffen, insbesondere an Süßstoffen und/oder Aromen, auf. Bei den üblichen Geschmacksstoffen, die in der erfindungsgemäßen Zubereitung enthalten sind, handelte es sich insbesondere um Ascorbinsäure, Weinsäure, Fumarsäure und/oder Zitronensäure, sowie Ascorbinsäure bevorzugt wird, da Ascorbinsäure zusätzlich noch als Vitamin C wirkt.

Wie bereits vorstehend erwähnt wurde, weist die erfindungsgemäße pharmazeutische Zubereitung eine feste Form auf und liegt vorzugsweise als Tablette oder Granulat vor. Selbstverständlich ist es jedoch auch möglich, die erfindungsgemäße Zubereitung als relativ grobkörniges Pulver aufzumachen.

Vorteilhafte Weiterbildungen der erfindungsgemäßen Zubereitung sind in den Unteransprüchen angegeben.

Die erfindungsgemäße Zubereitung wird anhand von Ausführungsbeispielen näher erläutert.

Nachfolgend werden bei den Ausführungsbeispielen die Begriffe Kompaktierung, Sprühtrocknung und Feuchtgranulierung verwendet.

Unter dem Begriff Kompaktierung wird ein Verfahren verstanden, bei dem der Wirkstoff bzw. das Wirkstoffgemisch mit einem in den Beispielen erwähnten konkreten Zuschlagsstoff bzw. ein Zuschlagsstoffgemisch, unter Umständen unter Zugabe von Wasser, mit einer geeigneten Vorrichtung, insbesondere einem Walzenkompaktor oder einer Tablettenpresse, kompaktiert wird. Anschließend wird das Kompaktat zerkleinert und ggf. getrocknet. Sollte das Kompaktat einen zu hohen Feinkornanteil, insbesondere unter 80 µm, aufweisen, so kann dieser Feinkornanteil durch Siebung entfernt werden.

Unter dem Begriff Sprühtrocknung wird ein Verfahren verstanden, bei dem der Wirkstoff bzw. das Wirkstoffgemisch in Wasser gelöst bzw. dispergiert wird. Hierbei werden die in den Ausführungsbeispielen konkret genannten Zuschlagsstoffe zugesetzt. Anschließend wird die Dispersion bzw. Lösung in einem Sprühturm im Warmluftstrom bei einer Produkttemperatur zwischen 30 °C und 120 °C getrocknet.

Unter dem Begriff Feuchtgranulierung wird ein Verfahren verstanden, bei dem der Wirkstoff bzw. das Wirkstoffgemisch in einer geeigneten Vorrichtung, insbesondere einem Mischer bzw. Wirbelschichtgranulator, unter Zugabe von in den Beispielen erwähnten Zuschlagsstoffen granuliert. Abhängig von dem jeweils ausgewählten Wirkstoff und den Zuschlagsstoffen kann das Granulat mit einem geeigneten Polymeren beschichtet werden.

Werden die der Sprühtrocknung, der Feuchtgranulierung und der Kompaktierung unterworfenen Produkte mit einem geeigneten Polymer, bei dem es sich bei den nachfolgenden Beispielen um das Produkt Eudragit E12,5 handelt, beschichtet, so ist dies bei den nachfolgend wiedergegebenen Ausführungsbeispielen durch Angabe des zuvor genannten Produktes gekennzeichnet.

Zu den nachfolgend in den Ausführungsbeispielen mit ihren Handelsnamen bezeichneten Bestandteilen Polyplasdone XL (GAF, USA), Aspartam (Nutra Sweet), Kollidon 25 (BASF), Acesulfam K (Hoechst) und Eudragit L (Röhm), Aerosil 200 (DEGUSSA), Biogapress 3326 (Gattefossé) sind jeweils in Klammern hinter dem einzelnen Bestandteil der Hersteller angegeben.

### Ausführungsbeispiel 1

### Herstellung einer Tablette mit dem Wirkstoff Ketoprofen

Eine Mischung, die
- 50 g: Ketoprofen,
- 5 g: Ethylcellulose und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 119 g: Glycin,
- 10 g: Polyplasdone XL,
- 1 g: Siliciumdioxid,
- 10 g: Aromastoffe,
- 1 g: Natriumchlorid,
- 2 g: Süßstoffe und
- 2 g: Magnesiumstearat
zugemischt. Die homogenisierte Mischung wurde zu 200 mg schweren Tabletten verpreßt. Die so hergestellte Tablette wurde als Tablette 1 bezeichnet.

### Ausführungsbeispiel 2

### Herstellung einer Tablette mit dem Wirkstoff Ibuprofen

Eine Mischung, die
- 400 g: Ibuprofen,
- 20 g: Gelatine und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 405 g: Glycin,
- 40 g: Polyplasdone XL,
- 20 g: Aromastoffe,
- 3 g: Siliciumdioxid,
- 1 g: Natriumchlorid,
- 2 g: Süßstoffe und
- 9 g: Magnesiumstearat
zugemischt. Die homogenisierte Mischung wurde zu 200 mg schweren Tabletten verpreßt. Die so hergestellte Tablette wurde als Tablette 2 bezeichnet.

### Ausführungsbeispiel 3

### Herstellung einer Tablette mit dem Wirkstoff Loperamid

Obwohl Loperamid ein hydrophiler Wirkstoff ist, wurde darauf verzichtet, diesen Wirkstoff mit einer hydrophoben Beschichtungsschicht zu versehen, da die Wirkstoffkonzentration in dieser Tablette relativ gering ist.

Eine Mischung, die
- 2 g: Loperamid-HCl,
- 85,7 g: Glycin,
- 5 g: Polyplasdone XL,
- 0,3 g: Siliciumdioxid,
- 5 g: Aromastoffe,
- 1 g: Süßstoffe und
- 1 g: Magnesiumstearat
wurde hergestellt. Aus der entsprechend homogenisierten Mischung wurden 100 mg schweren Tabletten gepreßt, die als Tabletten 3 bezeichnet wurden.

### Ausführungsbeispiel 4

### Herstellung einer Tablette mit dem Wirkstoff Acetylcystein

Eine Mischung, die
- 600 g: Acetylcystein,
- 25 g: Ethylcellulose und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 490 g: Glycin,
- 55 g: Polyplasdone XL,
- 1 g: Aspartam,
- 1 g: Saccharin-Natrium,
- 15 g: Aromastoffe,
- 3 g: Siliciumdioxid und
- 10 g: Magnesiumstearat
zugemischt. Die homogenisierte Mischung wurde zu 1.200 mg schweren Tabletten verpreßt. Die so hergestellte Tablette wurde als Tablette 4 bezeichnet.

### Ausführungsbeispiel 5

### Herstellung einer Tablette mit dem Wirkstoff Thymianextrakt

Eine Mischung, die
- 62,5 g: Thymian Trockenextrakt,
- 4 g: Eudragit L und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 160 g: Glycin
- 19 g: Polyplasdone XL,
- 0,5 g: Siliciumdioxid,
- 10 g: Aromastoffe,
- 40 g: Liquiritiae Succus Plv.,
- 1 g: Süßstoffe und
- 3 g: Magnesiumstearat
zugemischt. Die homogenisierte Mischung wurde zu 300 mg schweren Tabletten verpreßt. Die so hergestellte Tablette wurde als Tablette 5 bezeichnet. Das vorstehend genannte Wasser kann auch durch ein nicht toxisches Lösungsmittel für Eudragit ersetzt werden.

### Ausführungsbeispiel 6

### Herstellung einer Tablette mit dem als Extrakt aus der Echinacea angustifolia vorliegenden Wirkstoff

Eine Mischung, die
- 8 g: Extr. Rad. Echinaceae angustifoliae,
- 0,8 g: Methylhydroxypropylcellulose und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 120,7 g: Glycin,
- 8 g: Polyplasdone XL,
- 0,5 g: Siliciumdioxid,
- 8 g: Aromastoffe,
- 1 g: Süßstoffe und
- 3 g: Magnesiumstearat
zugemischt. Die homogenisierte Mischung wurde zu 150 mg schweren Tabletten verpreßt. Die so hergestellte Tablette wurde als Tablette 6 bezeichnet.

### Ausführungsbeispiel 7

### Herstellung einer Tablette mit dem Wirkstoff Carbocystein

Eine Mischung, die
- 375 g: Carbocystein,
- 11,6 g: Gelatine und
Wasser
enthielt, wurde granuliert und getrocknet. Nach Trocknung und Siebung des getrockneten Granulats über ein Sieb mit einer Maschenweite von 1 mm wurden die nachfolgenden Bestandteile
- 371,7 g: Glycin,
- 30 g: Polyplasdone XL,
- 5 g: Aerosil,
- 1 g: Aspartam pulv.,
- 1,5 g: Acesulfam K,
- 0,16 g: Orangenaroma und
- 4 g: Magnesiumstearat
zugemischt. Die homogenisierte Mischung wurde zu 810 mg schweren Tabletten verpreßt. Die so hergestellte Tablette wurde als Tablette 7 bezeichnet.

### Ausführungsbeispiel 8

### Herstellung einer Tablette mit dem Wirkstoff Paracetamol

Es wurde eine erste Mischung, die
- 81,95 g: Glycin und
- 2 g: Kollidon 25
enthielt, hergestellt. Desweiteren wurde eine zweite Mischung, die
- 106,95 g: Rhodapap NCR (mit Ethylcellulose beschichtetes Paracetamol),
- 6 g: Polyplasdone XL und
- 1 g: Aerosil 200
aufwies, erstellt.

Aus den Bestandteilen
- 0,2 g: Aspartam pulv.,
- 0,3 g: Acesulfam-K,
- 0,6 g: Aroma Typ Pfefferminz und
- 0,2 g: Natriumchlorid
wurde eine dritte Mischung hergestellt.

Die erste und die zweite Mischung wurden in einem geeigneten und in der Pharmazie üblichen Mischer miteinander vermischt. Zu dieser homogenisierten Mischung wurde dann die vorstehend aufgeführte dritte Mischung zugesetzt und entsprechend so lange vermischt, bis eine homogene Mischung entstand.

Zu dieser homogenen Mischung, bestehend aus der ersten, zweiten und dritten Mischung wurden
- 0,8 g: Magnesiumstearat
zugesetzt, wobei zuvor das Magnesiumstearat über ein Sieb mit einer Maschenweite von 0,3 mm ausgesiebt wurde. Nach einer Mischzeit von fünfzig Sekunden wurde die hierbei entstandene Mischung zu einer 1.000 mg schweren Tablette verpreßt, wobei diese Tablette als Tablette 8 bezeichnet wurde.

### Ausführungsbeispiel 9

### Herstellung einer Tablette mit einem Vitamingemisch-Wirkstoff

Eine Multivitamin-Mischung (142,8 g), bestehend aus
- 51,282 g: Ascorbinsäure (97,5 %),
- 15 g: Nicotinamid,
- 10 g: Vitamin E Acetat (50 %),
- 2 g: Pyridoxin HCl,
- 1,8 g: Riboflavin,
- 1,5 g: β-Carotin,
- 1,5 g: Thiaminnitrat,
- 0,3 g: Folsäure,
- 48,118 g: Palatinit,
- 0,5 g: Aerosil 200,
- 4 g: Biogapress 3326 und
- 6,8 g: Ethylcellulose
wurde granuliert und getrocknet. Nach Trocknung und Siebung des Granulates wurde das ausgesiebte Granulat mit einer Mischung aus
- 792,4 g: Glycin und
- 10 g: Kollidon 25
vermischt. Desweiteren wurde eine zweite Mischung, die
- 50 g: Polyplasdone XL,
- 5 g: Aerosil 200,
- 1 g: Aspartam pulv.,
- 1,5 g: Acesulfam K,
- 1 g: Natriumchlorid,
- 5 g: Pfirsicharoma,
- 2 g: Citronensäure und
- 4 g: Magnesiumstearat
enthielt, homogen dazugemischt. Aus dieser Mischung wurde dann eine 1.000 mg schwere Tablette gepreßt, die als Tablette 9 bezeichnet wurde.

### Ausführungsbeispiel 10

### Herstellung einer Tablette mit dem Wirkstoff Acetylsalicylsäure

Es wurde eine erste Mischung, die
- 85,897 kg: Glycin
- 2 kg: Kollidon 25 und
aufwies, erstellt.

Aus den Bestandteilen
- 105,263 kg: Aspirin NCR-P (entspricht 100 kg Acetylsalicylsäure, beschichtet mit Ethylcellulose),
- 5 kg: Weinsäure,
- 6 kg: Polyplasdone XL,
- 0,31 kg: Magnesiumstearat,
- 2,83 kg: Talcum mikr. und
- 2,7 kg: Hilfstoffe
wurde eine zweite Mischung hergestellt, wobei zunächst das Magnesiumstearat und das Talcum manuell vermischt wurde und erst zum Ende zu einer Vormischung, bestehend ausAspirin NCR-P, Weinsäure, Polyplasdone XL und den Hilfsstoffen, zugesetzt wurde. Die so hergestellte Gesamtmischung wurde dann zu einer 1.050 mg schweren Tablette verpreßt, die als Tablette 10 bezeichnet wurde.

### Ausführungsbeispiel 11

### Herstellung einer herkömmlichen Tablette mit dem Wirkstoff Aspirin

Es wurde eine erste Mischung, die
- 91,16 kg: Sorbitol und
- 2 kg: Kollidon 25
aufwies, erstellt.

Aus den Bestandteilen
- 100 kg: kristallines Aspirin,
- 5 kg: Weinsäure,
- 6 kg: Polyplasdone XL,
- 0,31 kg: Magnesiumstearat,
- 2,83 kg: Talcum mikr. und
- 2,7 kg: Hilfstoffe
wurde eine zweite Mischung hergestellt, wobei zunächst das Magnesiumstearat und das Talcum manuell vermischt wurde und erst zum Ende zu einer Vormischung, bestehend ausAspirin, Weinsäure, Polyplasdone XL und den Hilfsstoffen, zugesetzt wurde. Die so hergestellte Gesamtmischung wurde dann zu einer 1.050 mg schweren Tablette verpreßt, die als Tablette 11 bezeichnet wurde.

Nach den vorstehend in den Ausführungsbeispielen 1 bis 11 angegebenen Herstellungsvorschriften wurden jeweils weitere Tabletten I bis IX hergestellt, wobei diese weiteren Tabletten I bis IX jeweils kein Glycin aufwiesen. Vielmehr war hierbei das Glycin durch eine der Glycinmenge entsprechenden Menge an Sorbitol ersetzt.

Von den vorstehend genannten und nach den Ausführungsbeispielen 1 bis 11 hergestellten sowie von den Vergleichstabletten I bis IX, die kein Glycin aufwiesen, wurden die Zerfallszeiten bestimmt. Hierfür wurde eine modifizierte Apparatur nach DAB 10 verwendet.

Die modifizierte Apparatur bestand aus einem Gestell mit Siebboden, das sechs zylindrische Prüfröhrchen aus Glas enthielt. Die kopfseitig offenen Röhrchen waren fußseitig lediglich durch ein Netz aus rostfreiem Stahldraht verschlossen, so daß die Flüssigkeit frei in das Röhrchen eindringen konnte. Das Gestell wurde durch einen Motor regelmäßig vertikal auf- und abwärts bewegt, wobei die Geschwindigkeit so eingestellt wurde, daß das Gestell 28 bis 32 mal pro Minute um jeweils 50 bis 60 mm aufund abwärts bewegt wurde. Das Gestell wurde hierbei in einem 1-Liter-Becherglas angeordnet, wobei das Becherglas mit 350 ml gereinigtem Wasser gefüllt war. Die Positionierung des Gestells wurde so vorgenommen, daß die Röhrchen in der obersten Endstellung der Aufwärtsbewegung die Wasserschicht ganz verlassen hatten und in der untersten Endstellung der Abwärtsbewegung so weit in das Wasser eintauchten, daß alle, in den Röhrchen angeordneten Tabletten vollständig mit Wasser genetzt waren.

In jedem Röhrchen wurde jeweils eine Tablette angeordnet.

Die Temperatur des Wassers während der Messung wurde auf einen Wert zwischen 36 °C und 38 °C eingestellt.

Das Gestell wurde dann so lange auf- und abwärts bewegt, bis alle Prüflinge zerfallen waren. Die hierfür erforderliche Zerfallszeit wurde bestimmt, wobei in der nachfolgend wiedergegebenen Tabelle 1 jeweils die unterste und oberste Zerfallszeit, bestimmt durch sechs Messungen, angegeben sind.

**Tabelle 1**

| Zerfallszeiten der nach den Ausführungsbeispielen 1 bis 11 hergestellten Tabletten sowie der Tabletten I bis IX (ohne Zusatz von Glycin) | |
|---|---|
| Tablette gemäß Beispiel | Zerfallszeit in Sekunden |
| 1 | 8 - 15 |
| 2 | 6 - 14 |
| 3 | 3 - 6 |
| 4 | 10 - 16 |
| 5 | 8 - 10 |
| 6 | 6 - 9 |
| 7 | 9 - 12 |
| 8 | 8 - 12 |
| 9 | 12 - 18 |
| 10 | 3 - 8 |

| Vergleichstabletten | |
|---|---|
| 11 | 60 - 80 |
| I | 60 - 90 |
| II | 70 - 90 |
| III | 60 - 70 |
| IV | 90 - 120 |
| V | 90 - 100 |
| VI | 60 - 90 |
| VII | 80 - 90 |
| VIII | 70 - 90 |
| IX | 80 - 100 |

Es wurde ein Geschmacks- und Akzeptanzversuch mit 30 Personen durchgeführt, die über mehrere Tage eine handelsübliche Tablette (gemäß Ausführungsbeispiel 11) oder ein äußerlich identisches tablettenförmiges Mittel (gemäß Ausführungsbeispiel 10) eingenommen haben, das sich von dem bekannten Mittel dadurch unterschied, daß es zusätzlich die im Ausführungsbeispiel 10 angegebene Menge an Glycin enthielt. Beide Tablette konnten von den Personen in ihrem Äußeren nicht unterschieden werden. Alle Personen erhielten sowohl die handelsübliche Tablette als auch das vorstehend genannte tablettenförmige Mittel.

Übereinstimmend berichteten alle 30 Personen, wie angenehm und unproblematisch die Anwendung der mit Glycin versetzten Tablette (gemäß Ausführungsbeispiel 10) war.

Insbesondere bestätigten die Probanden übereinstimmend, daß im Vergleich zum herkömmlichen Analgetikum die mit Glycin versetzte Tablette im Mund während des Einnehmens und Kauens als aüßerst positiv und angenehm empfunden wurde. Das typische, bei der bekannten Tablette immer auftretende Verkleben von Tablettenbestandteilen an Gaumen, Zähnen und Zahnfleisch konnte bei der mit Glycin versetzten Tablette nicht festgestellt werden. Auch löste sich diese Tablette wesentlich schneller auf, was als sehr komfortabel empfunden wurde.

## Patentansprüche

1. Pharmazeutische, oral anwendbare Zubereitung, wobei die feste Zubereitung mindestens einen Wirkstoff, mindestens ein Sprengmittel sowie mindestens einen pharmazeutisch üblichen Zuschlagstoff aufweist, dadurch gekennzeichnet, daß die Zubereitung desweiteren mindestens einen, den Zerfall der Zubereitung im Mund oder in einer Flüssigkeit beschleunigenden Inhaltsstoff enthält, wobei dieser Inhaltsstoff eine wasserlösliche Aminosäure, ein wasserlösliches Aminosäurederivat und/oder ein wasserlösliches Salz einer Aminosäure ist.

2. Pharmazeutische Zubereitung nach Anspruch 1, dadurch gekennzeichnet, daß der Inhaltsstoff Glycin, ein Glycinderivat und/oder ein Glycinsalz ist.

3. Pharmazeutische Zubereitung nach Anspruch 1 oder 2, dadurch gekennzeichnet, daß der Inhaltsstoff Prolin, Hydroxyprolin und/oder Lysin, ein Salz und/oder ein Derivat davon ist.

4. Pharmazeutische Zubereitung nach einem der vorangehenden Ansprüche, dadurch gekennzeichnet, daß die Zubereitung den Inhaltsstoff in einer solchen Konzentration aufweist, daß die Zubereitung im Mund oder in einer Flüssigkeit innerhalb von einer Sekunde bis sechzig Sekunden, insbesondere innerhalb von einer Sekunde bis dreißig Sekunden, zerfällt.

5. Pharmazeutische Zubereitung nach Anspruch 4, dadurch gekennzeichnet, daß die Zubereitung den Inhaltsstoff in einer Konzentration zwischen 1 Gew.% und 90 Gew.%, insbesondere in einer Konzentration zwischen 20 Gew.% und 70 Gew.%, bezogen auf die anwendungsfertige Zubereitung, aufweist.

6. Pharmazeutische Zubereitung nach einem der vorangehenden Ansprüche, dadurch gekennzeichnet, daß das Sprengmittel Stärke, ein Stärkederivat, Cellulose, ein Cellulosederivat, Alginsäure, ein Alginsäurederivat, Casein, ein Caseinderivat und/oder ein unlösliches Polyvinylpyrrolidon ist.

7. Pharmazeutische Zubereitung nach einem der vorangehenden Ansprüche, dadurch gekennzeichnet, daß die Zubereitung das Sprengmittel in einer Konzentration zwischen 1 Gew.% und 50 Gew.%, insbesondere in einer Konzentration zwischen 3 Gew.% und 20 Gew.%, bezogen auf die anwendungsfertige Zubereitung, aufweist.

8. Pharmazeutische Zubereitung nach einem der vorangehenden Ansprüche, dadurch gekennzeichnet, daß die Zubereitung den mindestens einen Wirkstoff in Form von gleichmäßig in der festen Zubereitung verteilten Wirkstoffpartikeln aufweist.

9. Pharmazeutische Zubereitung nach einem der vorangehenden Ansprüche, dadurch gekennzeichnet, daß der mindestens eine Wirkstoff ein Analgetikum und/oder ein Antirheumatikum ist.

10. Pharmazeutische Zubereitung nach Anspruch 9, dadurch gekennzeichnet, daß der Wirkstoff ein Wirkstoff auf der Basis einer Pyrazolonverbindung, einer Salicylsäureverbindung, einer Phenylbutanzonverbindung, einer Paracetamolverbindung, einer Antranilsäureverbindung, einer Arylessigsäureverbindung und/oder einer Arylpropionsäureverbindung ist.

11. Pharmazeutische Zubereitung nach Anspruch 9 oder 10, dadurch gekennzeichnet, daß der Wirkstoff Paracetamol, Acetylsalicylsäure, Ketoprofen, Ibuprofen und/oder Diclofenac-Natrium ist.

12. Pharmazeutische Zubereitung nach einem der vorangehenden Ansprüche, dadurch gekennzeichnet, daß die Zubereitung den mindestens einen Wirkstoff in einer Konzentration zwischen 1 Gew.% und 50 Gew.%, insbesondere in einer Konzentration zwischen 15 Gew.% und 40 Gew.%, jeweils bezogen auf die anwendungsfertige Zubereitung, aufweist.

13. Pharmazeutische Zubereitung nach einem der vorangehenden Ansprüche, dadurch gekennzeichnet, daß die Zubereitung mindestens einen hydrophilen Wirkstoff enthält, der mit einer hydrophoben Beschichtungsschicht versehen und/oder in einer hydrophoben Matrix eingebettet ist.

14. Pharmazeutische Zubereitung nach Anspruch 13, dadurch gekennzeichnet, daß die hydrophobe Beschichtungsschicht und/oder die hydrophobe Matrix Schellack, Stearinsäure, Gelatine, Zein, Gummi arabicum, Cellulosederivate, polymere Acrylsäurederivate und/oder polymere Vinylacetate umfaßt.

15. Pharmazeutische Zubereitung nach einem der vorangehenden Ansprüche, dadurch gekennzeichnet, daß die feste Zubereitung als Zuschlagsstoff zwischen 0 Gew.% und 60 Gew.% Füll- und Bindemittel, jeweils zwischen 0 Gew.% und 10 Gew.% Povidon, Zucker, Zuckeralkohole, Polyethylenglycol sowie übliche Süßstoffe und/oder Aromen aufweist.

16. Pharmazeutische Zubereitung nach einem der vorangehenden Ansprüche, dadurch gekennzeichnet, daß die Zubereitung in Form einer Tablette oder eines Granulates vorliegt.
